# EUROPÄISCHE PATENTSCHRIFT

(11) **EP 3 000 446 B1**
(45) Veröffentlichungstag und Bekanntmachung des Hinweises auf die Patenterteilung: **12.02.2020**
(21) Anmeldenummer: 15173651.9
(22) Anmeldetag: 24.06.2015
(51) Int. Cl.: A61F 2/966, A61M 25/10, A61L 29/04, A61M 25/06

(54) **KATHETERSYSTEM UND VERFAHREN ZUR HERSTELLUNG EINES SOLCHEN**
CATHETER SYSTEM AND METHOD FOR PRODUCING SAME
SYSTÈME DE CATHETER ET SON PROCÉDÉ DE FABRICATION

(30) Priorität: 15.09.2014 US 201462050212 P; 15.09.2014 US 201462050211 P
(43) Veröffentlichungstag der Anmeldung: 30.03.2016
(73) Patentinhaber: Biotronik AG, 8180 Bülach (CH)
(72) Erfinder: Quint, Bodo, 72908 Dettighofen (DE)
(74) Vertreter: Randoll, Sören

(56) Entgegenhaltungen:
- US-A1- 2005 165 352
- US-A1- 2006 200 221

## Beschreibung

Die Erfindung betrifft ein Kathetersystem mit funktionalem Element am distalen Ende, einem mit seinem distalen Ende das funktionale Element umgebenden Zuführschlauch und einem am proximalen Ende des funktionalen Elements anstoßenden Schiebeelement zum Vorschieben des funktionalen Elements aus dem Zuführschlauch, um die Funktion des funktionalen Elements im Körper zu bewirken. Der im Lieferzustand verjüngenden und beim Herauschieben des Gefäßdilators expandierbaren Spitzenabschnitt des Zuführschlaugs ist aus einem temporär verklebten, unter Dehnung delaminierenden e-PTFE-Laminat geformt, wobei das e-PTFE-Laminat mit einem Klebstoff verklebt ist, welcher als functionswesentlichen Bestandteil eine Fettsäure aufweist. Die Erfindung betrifft des Weiteren ein Verfahren zur Herstellung eines solchen Systems.

Die Erfindung wird im Folgenden am Beispiel eines selbstexpandierenden Stents als funktionales Element beschrieben. Sie ist jedoch allgemein für Kathetersysteme geeignet, bei der ein funktionales Element von einem Zuführschlauch umgeben ist und mit Hilfe eines Schiebeelements, häufig als pusher bezeichnet, aus dem Zuführschlauch heraus geschoben wird. Als funktionale Elemente kommen hier beispielsweise bei mechanisch wirkenden Kathetersystemen Elemente in Frage, die dazu ausgebildet sind, Gewebeproben zu entnehmen, Gewebeablagerungen zu entfernen oder Gewebe zu penetrieren.
Im Rahmen dieser Anmeldung wird unter dem distalen Ende, z.B. des Kathetersystem, das Ende verstanden, welches vom Behandler entfernt liegt. Das proximale Ende des Kathetersystems ist entsprechend dem Behandler am nächsten.
Selbstexpandierende Stents, die ohne einen Ballonkatheter funktionieren, sind seit langen bekannt, und entsprechende Applikationssysteme sind im ständigen klinischen Einsatz. Im Hinblick auf die bevorzugt verwendeten Materialien zur Herstellung werden derartige Stents beispielsweise auch als "Nitinol-Stents" bezeichnet; sie werden unter anderem von der Anmelderin angeboten.

Es sind verschiedene Ausführungen von Kathetersystemen für selbstexpandierende Stents bekannt geworden, darunter Systeme ohne "inner lumen", die vorteilhafter Weise mit geringem Durchmesser realisierbar sind und eine Reduktion des Traumas beim Kathetereingriff ermöglichen und eine besonders hohe Flexibilität aufweisen. Bei solchen Systemen bildet der Stent selbst das Lumen für den Führungsdraht, und das distale Katheterende (Tip) muss mit dem Zuführschlauch verbunden bzw. als distaler Abschnitt desselben ausgebildet sein. Die Spitze wird über plastische Deformation geöffnet, wenn der Stent mittels des Schiebeelementes nach distal aus dem Zuführschlauch herausgeschoben wird. Die Spitze muss hinsichtlich ihrer mechanischen Eigenschaften sowohl den Anforderungen bei der Zuführung des Stents (Release) als auch beim Zurückziehen des Katheters genügen.

Vereinfacht ausgedrückt, bestehen derartige Systeme nur aus dem Zuführschlauch, der den selbstexpandierenden Stent, das Schiebeelement und den Führungsdraht umgibt. Der selbstexpandierenden Stents wird durch den Zuführschlauch in seinem komprimierten ("gecrimpten") Zustand gehalten. Durch das Schiebeelement wird der Stent aus dem Zuführschlauch heraus geschoben und expandiert ("dilatiert") in das umgebende Blutgefäß, um dieses zu erweitern oder zu stützen. Derartige Systeme verzichten auf den sonst üblichen Innenschlauch oder Innenschaft, der das Führungsdrahtlumen bildet bzw. den Führungsdraht umgibt, und den selbstexpandierenden Stent trägt.

Entsprechend haben solche Kathetersysteme der eingangs beschriebenen Art ohne Innenschaft den Vorteil, dass sie im Durchmesser reduziert werden können, wodurch in der Regel eine höhere Flexibilität erreicht wird und auf kleinere Hilfselemente (Einführhilfen und/oder Führungskatheter) gewechselt werden kann.

Bei einem Kathetersystem der eingangs genannten Art ohne Innenschaft muss die distale Katheterspitze zwangsläufig am Zuführschlauch befestigt sein. Die Katheterspitze rundet den Zuführschlauch ab und gewährleistet, dass das Gewebe beim Einführen des Kathetersystems und beim Vorschub zur eigentlichen Stelle im Körper, an der das funktionelle Element seine Funktion ausüben soll, nicht traumatisiert oder verletzt wird. Entsprechend soll eine derartige Spitze bei der Verformung durch das Freisetzen des funktionellen Elements nicht derart plastisch verformt werden, dass sie nach erfolgter Verformung potentielle traumatisch wird oder den Rückzug des Kathetersystems aus dem Körper behindert. Eine ideale elastische Deformation der Katheterspitze ist anzustreben, so dass das funktionelle Element unter stetiger Reibung durch die elastische Rückstellkraft der Katheterspitze freigesetzt werden muss. US 20050165352 A offenbart ferner ein Zuführsystem mit einem Stent und einer sich verjüngenden Spitze.

Der Erfindung liegt daher die Aufgabe zugrunde, ein Kathetersystem der genannten Art bereitzustellen, welches die speziellen Anforderungen an die mechanischen Eigenschaften der Spitze in den verschiedenen Phasen des Einsatzes des Systems in verbesserter Weise erfüllt. Des Weiteren soll ein Verfahren zur Herstellung eines solchen Kathetersystems angegeben werden.

Diese Aufgabe wird in ihrem Vorrichtungsaspekt durch ein Kathetersystem mit den Merkmalen des Anspruchs 3 sowie durch einen Zuführschlauch mit den Merkmalen des Anspruchs 1 und in ihrem Verfahrensaspekt durch ein Verfahren mit den Merkmalen des Anspruchs 10 gelöst. Zweckmäßige Fortbildungen des Erfindungsgedankens sind Gegenstand der jeweiligen abhängigen Ansprüche.

Die Erfindung geht von der Überlegung aus, dass bei einem Kathetersystem der genannten Art die Spitze in verschiedenen Einsatzphasen unterschiedliche geometrische Konfigurationen und mechanische Eigenschaften haben sollte: Im Lieferzustand sollte der Durchmesser des Systems (speziell des Zuführschlauches) sich zum distalen Ende hin verringern, es sollte also tatsächlich eine Art Spitze gebildet sein, welche zudem eine hinreichende Steifigkeit aufweist. In der Zuführphase sollte diese Spitze verformbar sein und idealerweise eine zweite geometrische Konfiguration annehmen, etwa zylindrisch oder trompetenförmig werden, und dann relativ weich sein und minimale Rückstellkräfte auf das frei zu setzende funktionelle Element ausüben. Um auch eine atraumatische Rückführung des Kathetersystems bzw. dessen Rückzug durch eventuelle Hilfssysteme nicht negativ zu beeinflussen, sollte sich auch das mechanische Verhalten des verformten Bereiches ändern. Die mechanischen Eigenschaften der nicht verformten Spitze sollen sich durch Zähigkeit und Biegsamkeit auszeichnen, während die verformte Spitze eine deutlich erhöhte Biegsamkeit und eine möglichst geringe Steifigkeit aufweisen soll. In beiden Zuständen muss die mechanische Integrität der Spitze gegeben bleiben. Die Oberfläche soll reibungsarm sein, um die bei Kontakt mit dem Körpergefäß dieses nicht zu reizen.

Von diesen Überlegungen ausgehend, schließt die Erfindung den Gedanken ein, den distalen Endabschnitt des Systems für die Liefersituation in einer solchen Weise spitzenartig und relativ steif auszubilden, dass bei der Einleitung und während der Zuführ- bzw. der Freisetzung des funktionellen Elements, insbesondere der Expansionsphase des Stents, in Folge einer vorbestimmten Materialumwandlung sowohl die Spitzen-Konfiguration als auch die mechanische Steifigkeit weitgehend aufgehoben werden. Des Weiteren schließt die Erfindung die Überlegung ein, dies mittels eines e-PTFE-Schichtmaterials zu erreichen, welches als solches bekannt ist und auch im medizinischen Bereich bereits eingesetzt wird.

E-PTFE ist als sehr weiches, elastisches, reibungsarmes und hydrophobes Material bekannt. Es lässt sich durch die thermische Verfahrenstechnik der Herstellung sehr gut auf elastische, plastische wie auf Festigkeitsanforderungen einstellen. Die bemerkenswerte Flexibilität im Vergleich zur Festigkeit beruht auf der Mikrostruktur von e-PTFE, die während Sinter und Verzugsprozessen im Herstellungsprozess entsteht.

Diese Mikrostruktur ermöglicht - im Gegensatz zu herkömmlichen PTFE-Bauteilen - eine hervorragende Klebbarkeit, wenn die Mikrostruktur des e-PTFE's durch den Klebstoff penetriert werden kann. Hiermit lässt sich leicht eine einfach auszuführende und zuverlässige Verbindung eines e-PTFE Schlauches zu einem verbindenden Schlauch, wie z. B. einem "Releaseschlauch" bzw. Zuführschlauch eines Katheters, herstellen.

Ein wesentlicher Aspekt der Erfindung besteht darin, dass der distale Endabschnitt des Zuführschlauches aus einem derart temporär verklebten e-PTFE-Laminat in einer Spitzen-Konfiguration besteht, dass der verklebte und durch die Verklebung sowohl in seine Form gebrachte als auch mechanische versteifte Verbund sich in der Zuführphase (wo er einer Dehnung unterliegt) mindestens teilweise delaminiert. Durch diese Delaminierung geht die vorgeprägte geometrische Form mindestens graduell verloren, d. h. die ursprüngliche Spitze weitet sich auf, und es bildet sich die weiter oben erwähnte wünschenswerte Zylinder-oder Trompeteform. Des Weiteren geht die mechanische Steifigkeit mindestens teilweise verloren, so dass die erwünschte Weichheit und Reduktion von auf den Stent einwirkenden Rückstellkräften eintritt.

In einer Ausführung der Erfindung ist der Spitzenabschnitt des Zuführschlauchs kegelstumpfförmig oder mit einem abgerundeten distalen Ende formgepresst. Hierbei kann das distale Ende sphärisch geformt sein, und eine konische Spitze kann speziell einen sphärischen Abschluss haben.

Es ist erfindungsgemäß vorgesehen, dass das e-PTFE-Laminat mit einem Klebstoff verklebt ist, welcher als funktionswesentlichen Bestandteil eine Fettsäure, insbesondere Stearinsäure, aufweist. Hierbei ist in Ausgestaltungen vorgesehen, dass der Klebstoff einen Ölsäurezusatz oder festigkeitserhöhenden polymeren Zusätze wie Polyvinylpyrrolidon oder ähnlichem aufweist. Die Kombination von Stearinsäure mit Ölsäure ist hierbei nur beispielhaft für sinnvolle Kombinationen von Klebstoffkomponenten für die temporäre Verklebung zu verstehen; außerdem können andere in der Fettsäurematrix lösbare Polymere außer PVP zur Eigenschafts-Verbesserung der funktionswesentlichen Fettsäure beitragen.

In einer weiteren Ausführung der Erfindung umfasst das Kathetersystem einen Stent, der derart ausgebildet ist, dass er unter Schubbelastung, beim Vorschieben aus dem Zuführschlauch, auf Block geht. Unter auf "Block gehen" wird im Rahmen dieser Anmeldung verstanden, dass der Stent derart ausgestaltet ist, dass sich einzelne Stentsegmente nicht überlappen oder über einander geschoben werden bzw. gegeneinander verkanten. Unter der Vielzahl bekannter und großteils auch kommerziell verfügbarer Stent-Konstruktionen findet der Fachmann solche, die diese Anforderung erfüllen und hierdurch vorteilhaft mit der vorgeschlagenen Gestaltung des distalen Endbereiches des Systems zusammenwirken können.

In sinnvollen geometrischen Konfigurationen ist vorgesehen, dass die Länge des Spitzenabschnitts im Bereich des 0,3-fachen bis 5-fachen, insbesondere des 0,6-fachen bis 2-fachen, Außendurchmessers des Zuführschlauchs liegt. Es ist allerdings zu verstehen, dass auch außerhalb dieser Relationen liegende Konfigurationen, bei denen die erwähnte temporäre, durch Dehnungs-Beanspruchung gezielt zerstörbare Verklebung des Endabschnittes vorliegt, zur Erfindung gehören.

In einer weiteren Ausführung ist der Spitzenabschnitt einstückig mit dem Zuführschlauch gebildet. Diese Ausführung ist sowohl technologisch besonders einfach zu realisieren als auch mechanisch stark belastbar. Grundsätzlich ist aber auch ein Verbund zwischen einer mit temporärer Verklebung versehenen Spitze aus einem ersten Material und einem Zuführschlauch aus einem zweiten Material als im Bereich der Erfindung liegend anzusehen. Bei einer solchen alternativen Ausführung lassen sich die Materialien für den langen Zuführschlauch und den kurzen Endabschnitt differenziert auswählen und an die jeweiligen Anforderungen anpassen.

Zur Herstellung des vorgeschlagenen Kathetersystems sind im Wesentlichen die herkömmlichen Verfahren geeignet, wobei die Realisierung der temporären und durch Dehnung aufhebbaren Verklebung des Zuführschlauch-Endabschnittes eine besondere Ausgestaltung erfordert. Abhängig von der Materialwahl des Klebstoffs und davon, ob der Endabschnitt einstückig mit dem übrigen Zuführschlauch gebildet ist oder nicht, ergibt sich eine Vielzahl von grundsätzlich möglichen Verfahrensvarianten.

Speziell für den Einsatz von Fettsäuren als funktionswesentlicher Bestandteil der Klebeverbindung wird eine Verfahrensführung vorgeschlagen, bei der ein schlauchförmig vorkonfiguriertes e-PTFE-Laminat bzw. e-PTFE Bauteil (vielschichtig aufgebaut) mit einer die Fettsäure (speziell Stearinsäure) enthaltenden Lösung bei erhöhter Temperatur getränkt und dann getrocknet wird. Später wird die gewünschte geometrische Form der Spitze durch Formpressen, ebenfalls bei erhöhter Temperatur, ausgebildet und zugleich eine Stauchung und somit mechanische Verdichtung des Materialsgefüges bewirkt. Nach dem Abkühlen liegt ein Spitzenabschnitt mit den für die Liefersituation des Applikationssystems gewünschten Eigenschaften vor.

In Ausgestaltungen dieses Verfahrens liegt die erste erhöhte Verfahrenstemperatur im Bereich zwischen 60°C und 90°C, insbesondere zwischen 70°C und 85°C und spezieller bei 80°C und die zweite Verfahrenstemperatur oberhalb des Schmelzpunktes der Fettsäure, insbesondere im Bereich zwischen 70°C und 90°C und spezieller im Bereich zwischen 75°C und 85°C.

Ein spezieller Vorteil dieser Applikationsmethode beruht auf der Beobachtung, dass aus Lösung getrocknet vorzugsweise ein kristalliner Niederschlag der Stearinsäure erhalten wird. Wird das e-PTFE mit dieser Lösung getränkt, bleibt es zunächst noch relativ flexibel. Unter Druck und Stauchung lässt sich solch ein getränkter Schlauch in die Form einer konischen Spitze komprimieren. Ab einer Temperatur beim Schmelzpunkt (69°C) wird die Stearinsäure aufgeschmolzen und bildet in Verbindung mit dem e-PTFE im Prinzip einen "Faserverbundwerkstoff", der zumindest sehr druckfest und reibungsarm ist. Unter Zugbelastung erfolgt dann aber - gegeben durch die geringe Festigkeit der Matrix - eine Delamination der Matrix, und je nach Belastung wird der "e-PTFE" Schlauch wieder freigesetzt. Dieser erhält annähernd seine Flexibilität zurück.

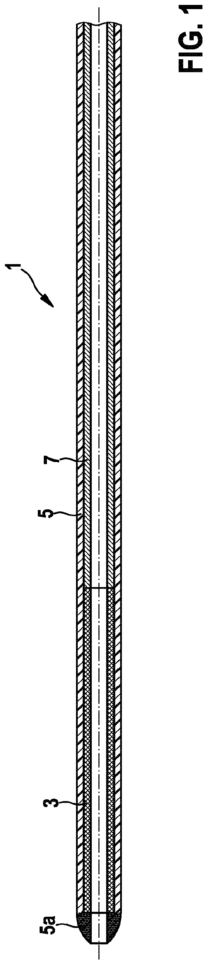

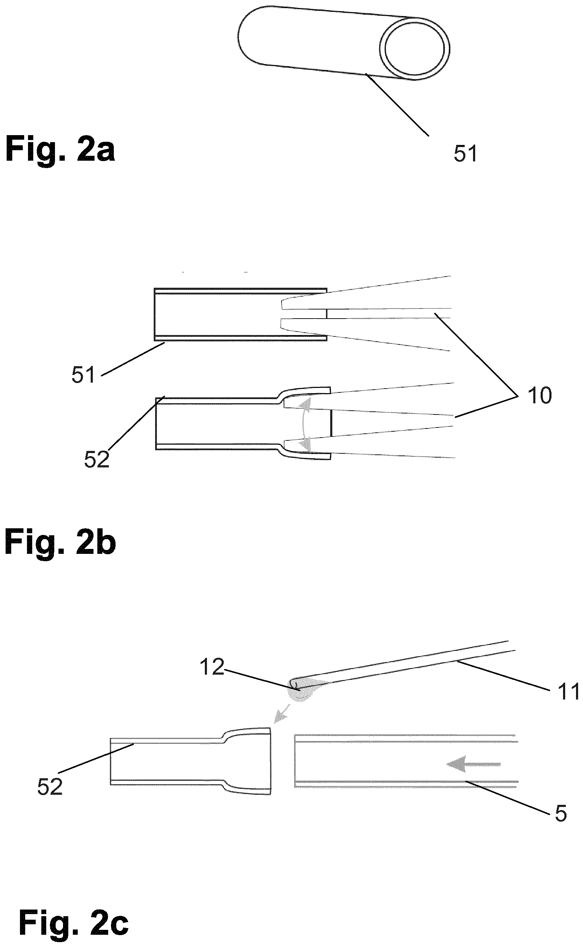

Vorteile und Zweckmäßigkeiten der Erfindung ergeben sich aus der nachfolgenden Beschreibung anhand des in Figur 1 dargestellten Ausführungsbeispiels der Erfindung. Die Figuren 2a-2f zeigen ein Ausführungsbeispiel eines Verfahrens zur Herstellung eines Zuführschlauches mit einer derartigen Spitze.

Es zeigen
- Fig. 1: ein erfindungsgemäßes Ausführungsbeispiel eines Kathetersystem zur Implantation eines selbstexpandierenden Stents und
- Fig. 2a-f: ein Ausführungsbeispiel zur Herstellung und Befestigung einer Spitze an einen Zuführschlauch gemäß der Erfindung

Figur 1 zeigt schematisch den distalen Endbereich eines Kathetersystems 1 eines selbstexpandierenden Stents 3. Der Stent 3 ist bevorzugt aus Nitinol und in dem in der Figur gezeigten Lieferzustand komprimiert in einem distalen Endabschnitt eines Zuführschlauches 5 untergebracht, in dem außerdem - proximal an das proximale Ende des Stents anstoßend - ein flexibles Schubrohr 7 als Schiebeelement zum Freisetzen des Stents untergebracht ist.

Der Zuführschlauch 5 ist aus einem e-PTFE-Laminat gebildet, und in einem distalen Endabschnitt 5a ist durch Tränkung mit einem Klebstoff auf Stearinsäure-Basis und Thermokompressions-Behandlung in der oben beschriebenen Weise ein temporär verklebter, verdichteter und gekrümmt chronisch zulaufender Spitzenbereich gebildet.

Dieser Spitzenbereich ist im Lieferzustand relativ steif. Er wird beim Vorschieben des Stents 3 mittels des Schubrohres 7 durch den Stent auf Dehnung beansprucht, und die Klebstoffmatrix wird aufgebrochen und der Materialverbund insofern delaminiert und in einen zylinder- bis trompetenförmigen Release-Zustand umkonfiguriert, der gegenüber dem Ausgangszustand auch mechanisch wesentlich weicher ist. Die hohe Gleitfähigkeit des e-PTFE erleichtert zusätzlich das Vorschieben des Stents, wie auch, nach dessen Zuführung, das Zurückziehen des Zuführschlauches aus dem entsprechenden Gefäß.

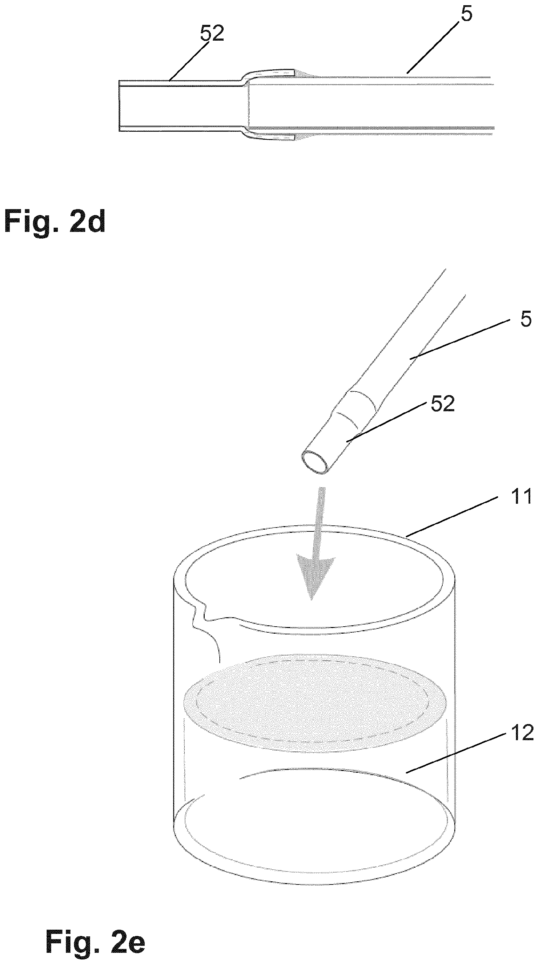

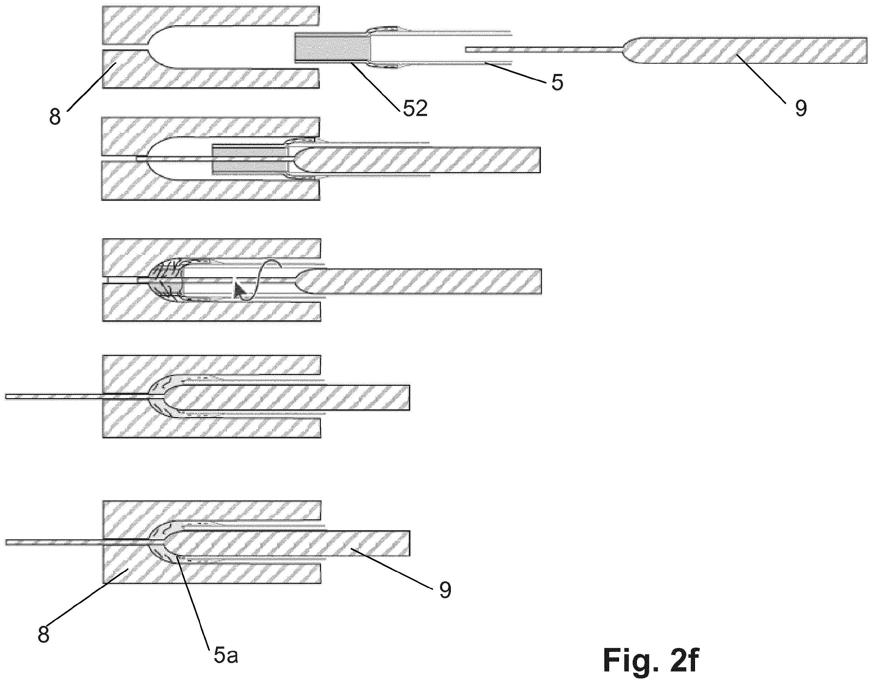

In den Figuren 2a bis 2f wird ein Ausführungsbeispiel der Herstellung eines erfindungsgemäßen Zuführschlauches für ein erfindungsgemäßes Kathetersystem gezeigt. Figur 2a zeigt ein zugeschnittenes Stück 51 eines e-PTFE Schlauches, welcher wie in Figur 2b gezeigt mit einem geeigneten Werkzeug 10 (beispielsweise eine Zange) an einer Seite aufgeweitet wird. Das aufgeweitete e-PTFE Schlauchstück 52 wird mittels eines geeigneten Stabes 11 mit einem Cyanacrylat-Kleber kontaktiert und auf das Ende eines Zuführschlauches 5 geschoben (Figur 2c), so dass eine Zuführschlauch-Spitze Vorform wie in Figur 2d entsteht. Diese Vorform aus e-PTFE Schlauchstück 52 und Zuführschlauch 5 wird in ein Gefäß 12 mit einer Stearinsäure enthaltenden Lösung 13 getaucht (Figur 2e), so dass das e-PTFE Schlauchstück 52 mit dieser Lösung beschichtet ist. Die Kombination aus beschichteten e-PTFE Schlauchstück 52 und Zuführschlauch 5 wird mit einem geeigneten Stempel 9 in eine erwärmbare Form 8 geschoben (Figur 2f). In der Form 8 wird die Kombination aus e-PTFE Schlauchstück 52 und Zuführschlauch 5 unter Bewegung bis oberhalb der Schmelztemperatur der Olefinmatrix erwärmt (in diesem Ausführungsbeispiel auf über 76°C) und zusammen gepresst (verdichtet). Nach anschließender Abkühlung entsteht derart ein erfindungsgemäßer Zuführschlauch 5 mit einem distalen Endabschnitt 5a das als Spitze im Sinne der Erfindung ausgebildet ist.

## Patentansprüche

1. Zuführschlauch für ein Kathetersystem mit einem sich im Lieferzustand verjüngenden und beim Herausschieben des Gefäßdilators expandierbaren Spitzenabschnitt, welcher aus einem temporär verklebten, unter Dehnung delaminierenden e-PTFE-Laminat geformt ist, wobei das e-PTFE-Laminat mit einem Klebstoff verklebt ist, welcher als funktionswesentlichen Bestandteil eine Fettsäure aufweist.

2. Zuführschlauch nach Anspruch 1, wobei der Spitzenabschnitt einstückig mit dem Zuführschlauch gebildet ist.

3. Kathetersystem mit funktionalen Element am distalen Ende, einem mit seinem distalen Ende das funktionale Element umgebenden Zuführschlauch nach einem der vorhergehenden Ansprüche und einem am proximalen Ende des funktionalen Elements anstoßenden Schiebeelement zum Vorschieben des funktionalen Elements aus dem Zuführschlauch.

4. Kathetersystem nach Anspruch 3, **dadurch gekennzeichnet, dass** das funktionale Element ein selbst-expandierender Stent ist.

5. Kathetersystem nach Anspruch 3 oder 4, **dadurch gekennzeichnet, dass** der Spitzenabschnitt des Zuführschlauchs kegelstumpfförmig oder mit einem abgerundeten distalen Ende formgepresst ist.

6. Kathetersystem nach einem der Ansprüche 3 bis 5, **dadurch gekennzeichnet, dass** der Klebstoff Stearinsäure aufweist.

7. Kathetersystem nach Anspruch 6, wobei der Klebstoff einen Ölsaure- oder festigkeitserhöhenden polymeren Zusatz von Polyvinylpyrrolidon oder ähnlichem aufweist.

8. Kathetersystem nach einem der vorangehenden Ansprüche, wobei die Länge des Spitzenabschnitts im Bereich des 0,3-fachen bis 5-fachen, insbesondere des 0,6-fachen bis 2-fachen, Außendurchmessers des Zuführschlauchs liegt.

9. Kathetersystem nach einem der vorangehenden Ansprüche, wobei der Spitzenabschnitt einstückig mit dem Zuführschlauch gebildet ist.

10. Verfahren zur Herstellung eines Kathetersystems nach einem der Ansprüche 3 - 9, wobei der Spitzenabschnitt des Zuführschlauchs aus einem schlauchförmig vorkonfigurierten e-PTFE-Laminat durch Tränken desselben mit einer Fettsäure, insbesondere Stearinsäure, enthaltenden, insbesondere alkoholischen, Lösung bei einer ersten gegenüber Raumtemperatur erhöhten Verfahrenstemperatur und späteres Formpressen des getränkten Spitzenabschnitts bei einer zweiten erhöhten Verfahrenstemperatur gebildet wird.

11. Verfahren nach Anspruch 10, wobei die erste erhöhte Verfahrenstemperatur im Bereich zwischen 60°C und 90°C, insbesondere zwischen 70°C und 85°C und spezieller bei 80°C und die zweite Verfahrenstemperatur oberhalb des Schmelzpunktes der Fettsäure, insbesondere im Bereich zwischen 70°C und 90°C und spezieller im Bereich zwischen 75°C und 85°C, liegt.

## Claims

1. A feed tube of a catheter system with a tip portion that tapers in the delivered state and expands when the functional element is slid out, which tip portion is formed from a temporarily glued e-PTFE laminate that delaminates when stretched, **characterized in that** the e-PTFE laminate is glued using an adhesive that has a fatty acid as a component essential to function.

2. The feed tube according to Claim 1, wherein the tip portion is formed integrally with the feed tube.

3. A catheter system with functional element at the distal end, a feed tube according to one of the preceding claims which, with the distal end thereof, surrounds the functional element, and a sliding element striking against the proximal end of the functional element in order to push forward the functional element from the feed tube.

4. The catheter system according to Claim 3, **characterized in that** the functional element is a self-expanding stent.

5. The catheter system according to Claim 3 or 4, **characterized in that** the tip portion of the feed tube is in the shape of a truncated cone or is molded with a rounded distal end.

6. The catheter system according to one of Claims 3 to 5, **characterized in that** the adhesive includes stearic acid.

7. The catheter system according to Claim 6, wherein the adhesive comprises an oleic acid- or strength-increasing polymeric additive of polyvinylpyrrolidone or the like.

8. The catheter system according to one of the preceding claims, wherein the length of the tip portion is in the range of 0.3 times to 5 times, in particular 0.6 times to 2 times, the outer diameter of the feed tube.

9. The catheter system according to one of the preceding claims, wherein the tip portion is formed integrally with the feed tube.

10. A method for producing a catheter system according to one of Claims 3-9, wherein the tip portion of the feed tube is formed from an e-PTFE laminate by saturation thereof with a solution, in particular an alcohol solution, containing the fatty acid (specifically stearic acid) at a first method temperature that is elevated compared with room temperature and subsequent molding of the saturated tip portion at a second increased method temperature.

11. The method according to Claim 10, wherein the first elevated temperature lies in the range between 60°C and 90°C, in particular between 70°C and 85°C and especially at 80°C and the second method temperature is above the melting point of the fatty acid, in particular in the range between 70°C and 90°C and especially in the range between 75°C and 85°C.

## Revendications

1. Tube d'alimentation pour système de cathéter avec une section en pointe, s'amenuisant dans un état de livraison et se dilatant lors du coulissement de sortie du dilatateur de vaisseau, laquelle est formée à base d'un stratifié de e-PTFE collé temporairement se délaminant sous tension, où le stratifié de e-PTFE est collé avec un adhésif lequel présente un acide gras en tant que constituant indispensable à la fonction.

2. Tube d'alimentation selon la revendication 1, dans lequel la section en pointe est formée d'un seul tenant avec le tube d'alimentation.

3. Système de cathéter doté d'un élément fonctionnel à l'extrémité distale, d'un tube d'alimentation selon l'une des revendications précédentes entourant l'élément fonctionnel avec son extrémité distale et d'un élément coulissant se mettant en butée à l'extrémité proximale de l'élément fonctionnel pour l'avancée de l'élément fonctionnel hors du tube d'alimentation.

4. Système de cathéter selon la revendication 3, **caractérisé en ce que** l'élément fonctionnel est un stent auto-expansible.

5. Système de cathéter selon la revendication 3 ou la revendication 4, **caractérisé en ce que** la section en pointe du tube d'alimentation est en forme de cône tronqué ou est moulée par compression avec une extrémité distale arrondie.

6. Système de cathéter selon l'une des revendications 3 à 5, **caractérisé en ce que** l'adhésif présente de l'acide stéarique.

7. Système de cathéter selon la revendication 6, dans lequel l'adhésif présente un additif à base d'acide oléique ou de polymères de polyvinyl pyrrolidone ou similaire augmentant la résistance.

8. Système de cathéter selon l'une des revendications précédentes, dans lequel la longueur de la section en pointe se situe dans la plage de 0,3 fois à 5 fois, notamment de 0,6 fois à 2 fois, le diamètre extérieur du tube d'alimentation.

9. Système de cathéter selon l'une des revendications précédentes, dans lequel la section en pointe est formée d'un seul tenant avec le tube d'alimentation.

10. Procédé de fabrication d'un système de cathéter selon l'une des revendications 3 à 9, dans lequel la section en pointe du tube d'alimentation est formée à base d'un stratifié de e-PTFE préformé en forme de tube par une trempe de celui-ci dans une solution, notamment alcoolique, contenant un acide gras, notamment l'acide stéarique, à une première température de procédé augmentée par rapport à la température ambiante et par un moulage par compression ultérieur de la section en pointe trempée à une seconde température de procédé augmentée.

11. Procédé selon la revendication 10, dans lequel la première température de procédé augmentée se situe dans la plage entre 60 °C et 90 °C, notamment entre 70 °C et 85 °C et spécialement à 80 °C et la seconde température de procédé se situe au-dessus du point de fusion de l'acide gras, notamment dans la plage entre 70 °C et 90 °C et spécialement dans la plage entre 75 °C et 85 °C.
